(19) Europäisches Patentamt
European Patent Office
Office européen des brevets

(11) EP 1 025 830 A2

(12) **EUROPÄISCHE PATENTANMELDUNG**

(43) Veröffentlichungstag:
**09.08.2000 Patentblatt 2000/32**

(51) Int. Cl.[7]: **A61K 6/087**

(21) Anmeldenummer: **00250010.6**

(22) Anmeldetag: **13.01.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **01.02.1999 DE 19905093**

(71) Anmelder: **IVOCLAR AG**
**9494 Schaan (LI)**

(72) Erfinder:
- **Moszner, Norbert**
  **9492 Eschen (LI)**
- **Jocham, Daniel**
  **8564 Krottendorf 251 (AT)**
- **Stelzer, Franz**
  **8020 Graz (AT)**
- **Rheinberger, Volker**
  **9490 Vaduz (LI)**

(74) Vertreter: **UEXKÜLL & STOLBERG**
**Patentanwälte**
**Beselerstrasse 4**
**22607 Hamburg (DE)**

## (54) Schrumpfungsarme Dentalmaterialien

(57) Die Erfindung betrifft Zusammensetzungen enthaltend (i) mindestens ein bicyclisches Ringsystem mit 6 bis 17 Kohlenstoff-, gegebenenfalls einem Heteroatom und mindestens einer endocyclischen Doppeldbindung; und (ii) mindestens einen Initiator für die ringöffnende Metathese-Polymerisation; wobei das bicyclische Ringsystem nicht mit Methacrylatgruppen substituiert ist. Die Zusammensetzungen eignen sich besonders zur Herstellung von Dentalmaterialien.

EP 1 025 830 A2

**Beschreibung**

Die Erfindung betrifft Zusammensetzungen, die mindestens ein ungesättigtes bicyclisches Ringsystem und einen Initiator für die ringöffnende Metathese-Polymerisation enthalten sowie deren Verwendung als Dentalmaterial.

Es ist bekannt, daß bei der Polymerisation von Monomeren oder Monomermischungen in der Regel eine mehr oder weniger stark ausgeprägte Volumenkontraktion stattfindet (vgl. R.R. Sadhir, R.M. Luck, Expanding Monomers - Synthesis, Characterization and Application; CRC Press, Boca Raton 1992, Seiten 3ff). Das Schrumpfen ist auf die Ausbildung kovalenter Bindungen zwischen den Monomermolekülen bei der Polymerisation zurückzuführen, wodurch der Abstand zwischen den Molekülen verringert wird. Im unpolymerisierten Zustand werden die Moleküle überwiegend durch Van der Waals-Kräfte zusammengehalten, welche einen größeren intermolekularen Abstand zur Folge haben.

Bei der Herstellung von Formteilen wirkt sich der Polymerisationsschrumpf sehr nachteilig auf die Dimensionsstabilität und die mechanischen Eigenschaften der Formkörper aus. Bei Adhäsiven und Klebeverbindungen beeinträchtigt der Polymerisationsschrumpf die Haftungseigenschaften und die Verbundfestigkeit, was bei Dentalmaterialien die Haftung zwischen Restaurationsmaterial und der natürlichen Zahnsubstanz verschlechtert. Es kommt zur Ausbildung von Spalten, welche die Bildung von Sekundärkaries begünstigen.

Zur Verringerung des Polymerisationsschrumpfes von Dentalmaterialien wurde der Einsatz von hochmolekularen Monomeren oder Präpolymeren, die Zugabe von inerten, porösen oder expandierenden Füllstoffen oder Gas freisetzenden Systemen beschrieben.

Weiterhin ist die Verwendung von Monomeren bekannt, die sich durch Ringöffnungspolymerisation polymerisieren lassen. Hierbei wird für jede neu geknüpfte kovalente Bindung eine bereits bestehende kovalente Bindung gespalten, so daß der Einfluß der Polymerisation auf die Abstände der Monomermoleküle minimiert wird.

Die US 4 387 215 offenbart Spiroorthoester, Spiroorthocarbonate und polycyclische Ketallactone, welche sich ringöffnend polymerisieren lassen und welche bei der Polymerisation keinen Schrumpf und teilweise sogar eine Expansion zeigen sollen.

Die WO 94/00501 beschreibt ein Verfahren zur Herstellung von Polymeren, welche sich wiederholende Spiroorthoestergruppen enthalten und sich zur Herstellung von Dentalmaterialien eignen.

Die DE 195 06 222 A1 offenbart kationisch polymerisierbare, schrumpfungsarme Materialien für medizinische Anwendungen und dentale Zwecke auf der Basis von Oxetan- und Oxacyclobutan-Derivaten.

Die DE 44 39 485 C2 betrifft bicycloaliphatische 2-Methylen-1,3-dioxepane, die sich radikalisch und kationisch polymerisieren lassen.

In der US 5, 665 839 werden radikalisch polymerisierbare Oxathiepane beschrieben, die die Herstellung von Polymeren mit Ester, Amid oder Thioestergruppen erlauben.

Die DE 196 12 004 A1 offenbart multifunktionelle Vinylcyclopropan-Derivate, die sich insbesondere zur Herstellung von Dentalmaterialien eignen. Die Vinylcyclopropane sind radikalisch polymerisierbar und bilden Polymere ohne hydrolytisch spaltbare Gruppen in der Hauptkette.

Diesen bekannten Monomeren ist gemeinsam, daß ihre Synthese aufwendig und teuer ist. Darüber hinaus sind Monomere wie Spiroorthocarbonate, Spiroorthoester oder 2-Methylen-1,3-dioxepane feuchtigkeitsempfindlich und weisen bei Raumtemperatur und in Gegenwart von $SiO_2$ oder silikatischen Füllstoffen nur eine begrenzte Stabilität auf.

Die DE 196 08 316 A1 betrifft durch (Meth)acrylatgruppen substituierte Norbornenyl-Derivate, die durch ringöffnende Metathese-Polymerisation (RÖMP) polymerisiert werden können und sich besonders zur Herstellung von Dentalmaterialien eignen.

Obwohl durch die im Stand der Technik beschriebenen Monomere die Schrumpfung von Dentalmaterialien bei der Polymerisation verringert werden konnte, stellt dieser nach wie vor eines der Hauptdefizite von Kompositwerkstoffen dar (A. Peutzfeldt, Eur. J. Oral Sci. **105** (1997) 97-116). Zudem fehlt es an schrumpfungsarmen Monomeren, die leicht und damit kostengünstig zugänglich sind und gleichzeitig eine ausreichende chemische Stabilität aufweisen.

Der Erfindung liegt die Aufgabe zugrunde, Zusammensetzungen bereitzustellen, die einfach herstellbar, bei Raumtemperatur und in Gegenwart herkömmlicher Dentalfüllstoffe lagerstabil und feuchtigkeitsunempfindlich sind und die nur einen geringen Polymerisationsschrumpf zeigen.

Diese Aufgabe wird durch Zusammensetzungen gelöst, die

(i) mindestens ein bicyclisches Ringsystem mit 6 bis 17, vorzugsweise 7 bis 10 Kohlenstoffatomen, gegebenenfalls einem oder mehreren Heteroatomen und mindestens einer endocyclischen Doppelbindung; und

(ii) mindestens einen Initiator für die ringöffnende Metathese-Polymerisation enthalten.

Unter bicyclischen Ringsystemen werden im Rahmen dieser Erfindung vorzugsweise Brücken-Ringsysteme verstanden.

Die erfindungsgemäß verwendeten bicyclischen Ringsysteme weisen keine (Meth)acrylatgruppen auf, so daß die Härtung der Zusammensetzungen ausschließlich durch ringöffnende Metathesepolymerisation (RÖMP) erfolgt. Darüber hinaus beeinträchtigen (Meth)acrylatgruppen die Stabilität der Monomeren und können bei der Vakuumdestillation beispielsweise zur Polymerisation führen, was die Reindarstellung erschwert. Desweiteren enthalten die bicyclischen Ringsystem vorzugsweise auch keine Vinyl- oder Allylgruppen da diese bei der RÖMP als Kettenüberträger fungieren und diese so negativ beeinflussen.

Außerdem enthalten die bicyclischen Ringsysteme vorzugsweise auch keine Carboxyl- oder Hydroxylgruppen da diese zur Bildung von kristallinen oder hochviskosen Verbindungen führen und darüber hinaus nach der Polymerisation durch Wasseraufnahme eine Quellung des Polymeren bewirken können.

Die erfindungsgemäßen Zusammensetzungen enthalten vorzugsweise bicyclische Ringsysteme mit 1 bis 2 endocyclischen Doppelbindungen. Besonders bevorzugt sind carbocyclische Ringsysteme sowie sauerstoffhaltige Heterocyclen. Heterocyclen mit nur einem Heteroatom sind bevorzugt.

Als besonders geeignet erwiesen sich carbocyclische und heterocyclische Bicyclo[x.y.z]kohlenwasserstoffe, wobei x, y und z Werte von 1 bis 6 aufweisen können. Vorzugsweise ist x gleich 2, y gleich 2 und z gleich 1.

Bevorzugte Vertreter dieser Verbindungsklasse sind Bicyclo[2.2.1]heptenderivate, insbesondere solche gemäß den folgenden Formeln I, II und III,

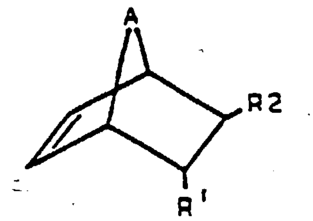

I

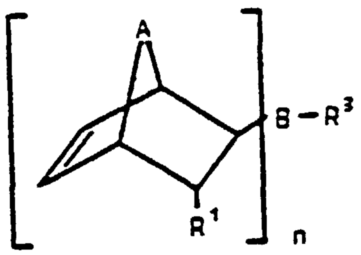

II

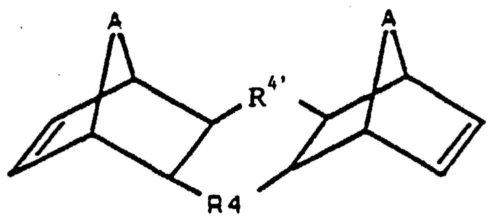

III

in denen n, A, B, $R^1$, $R^2$, $R^3$, $R^4$ und $R^{4'}$ unabhängig voneinander die folgenden Bedeutungen haben:

A = $-CH_2-$ oder -O-;

$R^1$, $R^2$ = -H; $C_1$- bis $C_6$-Alkyl, vorzugsweise $C_1$- bis $C_3$-Alkyl, insbesondere Methyl oder Ethyl; $-C(=O)-OR^5$; $-O-C(=O)-R^5$; $-CH_2-O-C(=O)-R^5$; wobei $R^5$ für -H, $C_1$- bis $C_6$-Alkyl oder Benzyl, vorzugsweise $C_1$- bis $C_3$-Alkyl, insbesondere Methyl oder Ethyl steht;
oder $R^1$ und $R^2$ bilden zusammen mit den Atomen, an die sie gebunden sind, ein ankondensiertes alicyclisches oder aromatisches Ringsystem mit 5 bis 12 Kohlenstoffatomen, vorzugsweise 5 bis 7 Kohlenstoffatomen, das seinerseits durch $C_1$- bis $C_6$-Akylgruppen oder Benzyl, vorzugsweise $C_1$- bis $C_3$-Alkyl,

insbesondere Methyl oder Ethyl, substituiert sein kann;

B = $-CH_2-$ oder entfällt;

n = eine ganze Zahl von 1 bis 4, vorzugsweise 1 bis 3;

$R^3$ = n-fach substituiertes $C_1$- bis $C_{10}$-Alkylen, vorzugsweise $C_1$- bis $C_6$-Alkylen, insbesondere $C_1$- bis $C_3$-Alkylen; $C_6$-bis $C_{10}$-Arylen, vorzugsweise Phenylen oder Naphthylen; -O-C(=O)-Phenylen-C(=O)-O-, vorzugsweise -O-C(=O)-(m- oder p-Phenylen)-C(=O)-O-; $(-C(=O)-)_4(C_6H_2)$; 2,4,6-Trioxo-1,3,5-Triazinyl; $-O-C(=O)-(CH_2)_m-C(=O)-O-$; $-O-C(=O)-NH-(CH_2)_m-NH-C(=O)-O-$; wobei m = 1 bis 10, vorzugsweise 1 bis 8, besonders bevorzugt 1 bis 6 ist;

$R^4$, $R^{4'}$ = $C_1$- bis $C_{20}$-Alkylen, vorzugsweise $C_1$- bis $C_{12}$-Alkylen, insbesondere $C_1$- bis $C_3$-Alkylen; eine chemische Bindung, -O- oder $R^4$ und $R^{4'}$ bilden zusammen einen $>CH-CH_2-CH<$ Rest.

sowie dazu stereoisomere Verbindungen und beliebige Mischungen dieser Substanzen.

Die Formel II deckt dabei auch die beiden Stellungsisomeren

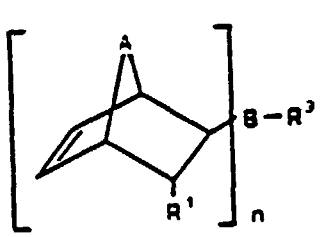

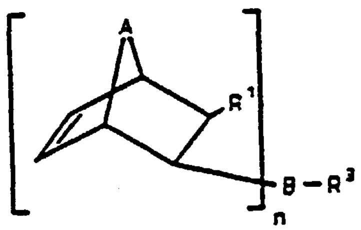

und die Formel III Stellungsisomere

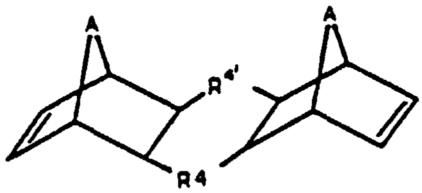

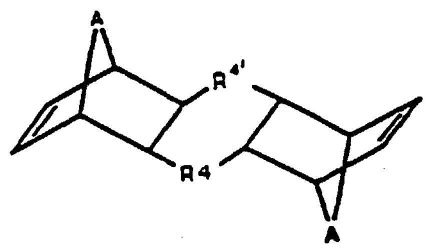

ab.

Die Reste $R^1$, $R^2$, $R^3$, $R^4$ und $R^{4'}$ können endo- oder exo-ständig gebunden sein. Typischerweise liegen die bicyclischen Verbindungen gemäß den Formeln I bis III in Form von stereoisomeren Gemischen, besonders als Racemate vor.

Die Reste $R^1$, $R^2$, $R^3$, $R^4$ und $R^{4'}$ können substituiert oder vorzugsweise unsubstituiert sein. Die gegebenenfalls vorhandenen Substituenten sind insbesondere Halogen, $C_1$- bis $C_{12}$-Alkoxy, $-N^+-(C_1$-bis $C_{12}$-Alkyl$)_3$, vorzugsweise $-N^+-(C_1$- bis $C_3$-Alkyl$)_3$, $-O-P=O(OH)_2$ oder $-P=O(OH)_2$. Besonders bevorzugte Substituenten sind $C_1$- bis $C_4$-Alkoxy und $-P=O(OCH_3)_2$. Sind mehrere Substituenten an den Resten vorhanden, können diese unabhängig voneinander gewählt werden.

Im Fall der Verbindungen gemäß Formel II ist der Rest $R^3$ n-fach, d.h. 1- bis 4-fach mit dem in der Klammer stehenden Norbornenyl-Rest substituiert.

Für die oben angegebenen Variablen der Formeln I, II und III existieren die folgenden unabhängig voneinander wählbaren bevorzugten Definitionen:

A = $-CH_2-$;

$R^1$, $R^2$ = -H; -C(=O)-$OCH_3$; -O-C(=O)-$CH_3$; -$CH_2$-O-C(=O)-$CH_3$;
oder $R^1$ und $R^2$ bilden zusammen mit den Kohlenstoffatomen, an die sie gebunden sind, ein ankondensiertes alicyclisches oder aromatisches Ringsystem mit 5 bis 7 Kohlenstoffatomen oder einen sauerstoffhaltigen Heterocyclus mit 4 Kohlenstoffatomen und einem Sauerstoffatom;

B = -$CH_2$- oder entfällt;

n = eine ganze Zahl von 1 bis 3;

$R^3$ = 1 bis 3-fach substituiertes $C_1$- bis $C_6$-Alkylen, insbesondere $C_1$- bis $C_3$-Alkylen; -O-C(=O)-(Phenylen)-C(=C)-C(=O)-O-; wobei m = 1 bis 6 ist;

$R^4$, $R^{4'}$ = $C_1$- bis $C_{12}$-Alkylen, insbesondere $C_1$- bis $C_3$-Alkylen, eine chemische Bindung oder -O-.

Bevorzugte Verbindungen sind demgemäß solche, bei denen mindestens eine der Variablen der Formeln I, II und III die vorstehend beschriebene bevorzugte Definition aufweist. Ganz besonders bevorzugt sind naturgemäß solche Verbindungen, bei denen mehrere oder alle Variablen den bevorzugten Definitionen entsprechen.

Ganz besonders bevorzugte bicylische Ringsysteme sind Bicyclo[2.2.1]hept-2-en (2-Norbornen) und davon abgeleitete Derivate wie 7-Oxa-bicyclo[2.2.1]hept-2-en, Bicyclo[2.2.1]hept-5-en-2,3-dicarbonsäuredimethylester, 7-Oxa-bicyclo[2.2.1]hept-5-endicarbonsäurediethylester, 5-Norbornen-2-methylester sowie 5-Norbornen-2-yl-ester von Mono-, Di- und Multicarbonsäuren oder die Umsetzungsprodukte von 5-Norbornen-2-methanol oder 5-Norbornen-2-ol mit Mono- oder Diisocyanaten.

Verbindungen, die mehrere Norbornengruppen enthalten führen bei der Polymerisation zu Polymernetzwerken und können deshalb als RÖMP-Vernetzer bezeichnet werden.

Für besonders bevorzugte bicyclische Ringsysteme werden nachstehend die entsprechenden Strukturformeln angegeben, wobei die Formeln auch für die entsprechenden Stellungsisomeren stehen, die sich durch den Austausch der Substituenten $R^1$, $R^2$ und $R^3$ ergeben.

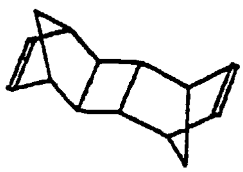
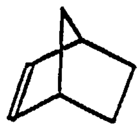
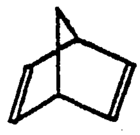

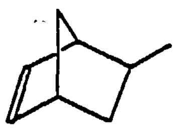
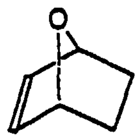
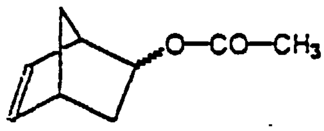

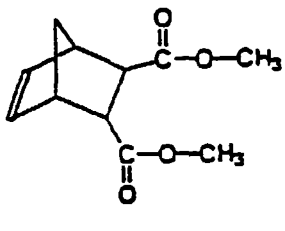
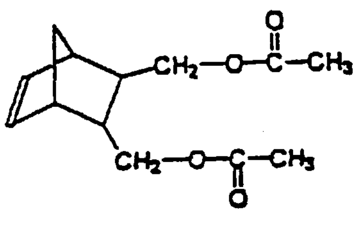

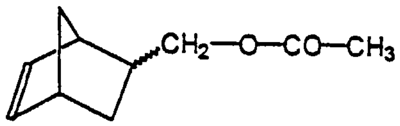
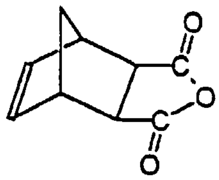
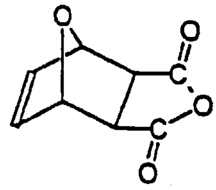

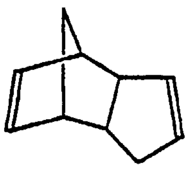
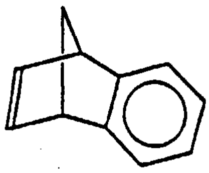
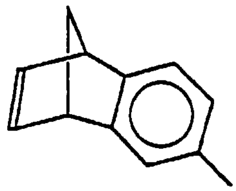
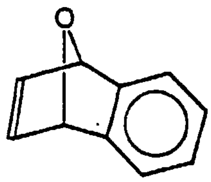

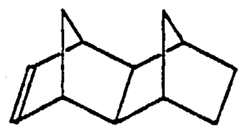
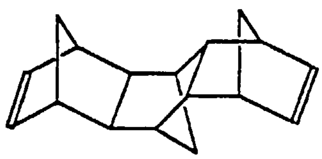
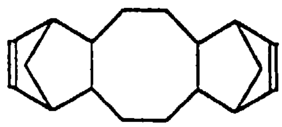

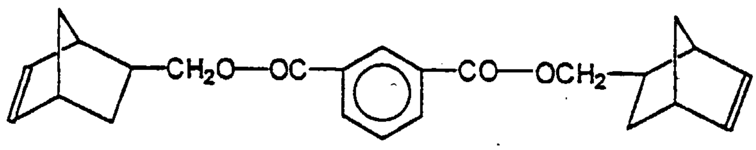

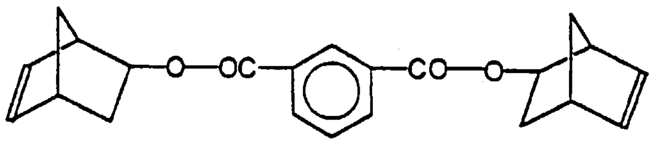

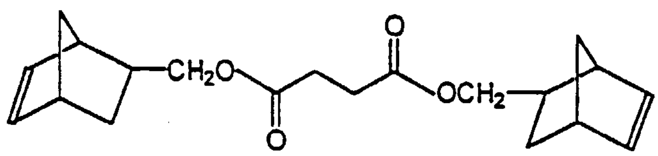

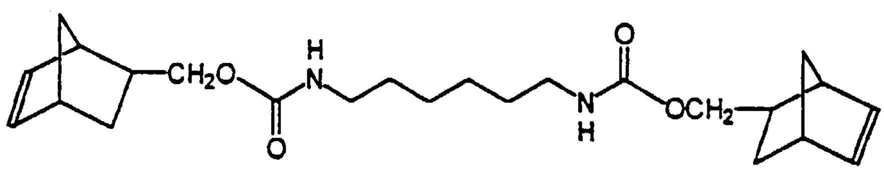

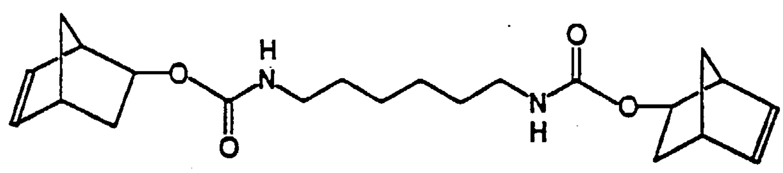

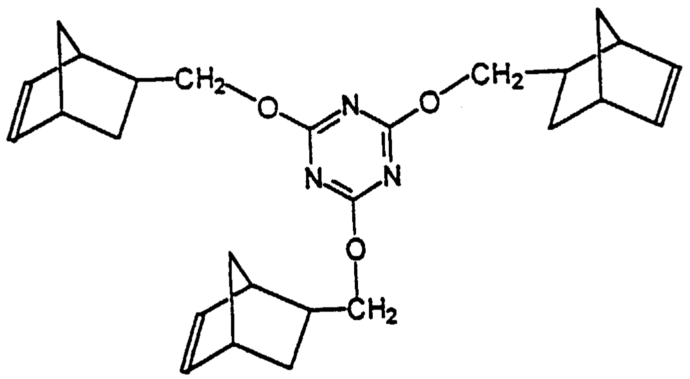

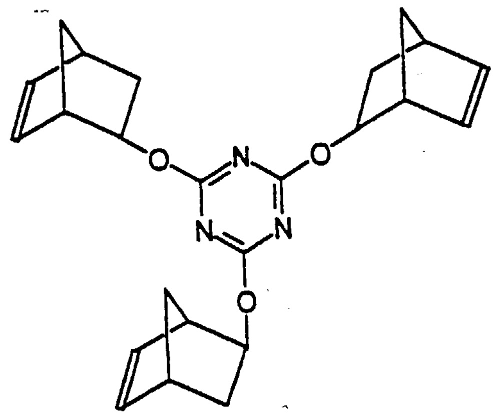

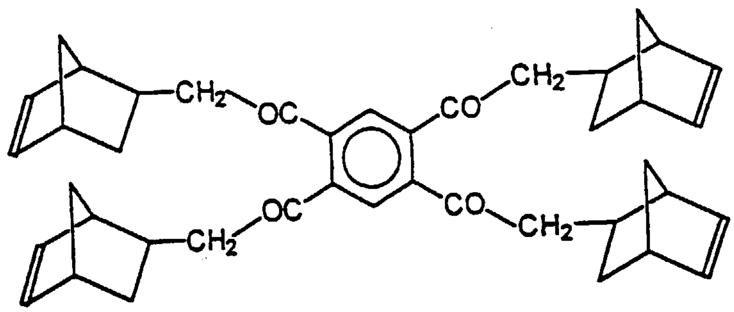

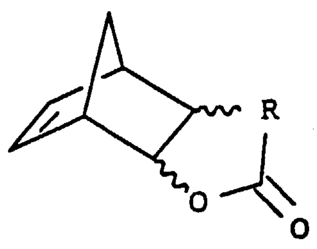

mit -R- = $-(CH_2)_n-$ wobei n gleich 1 bis 4 ist und der O und R enthaltende Ring endo- oder exo-ständig zum Norbornensystem ist Die aufgeführten bicyclischen Ringsysteme sind durch die bekannten Cyclisierungsreaktionen wie z.B. Cycloadditionen einfach zugänglich. Sie sind bei Raumtemperatur und in Gegenwart von herkömmlichen Dentalfüllstoffen stabil und nicht feuchtigkeitsempfindlich.

Neben den oben definierten bicyclischen Ringsystemen enthalten die Zusammensetzungen einen Initiator

für die ringöffnende Metathese-Polymerisation, vorzugsweise einen Metallcarben-Komplex wie z.B. einen Ru-, W- und/oder Mo-Carben-Komplex (vgl. R.R. Schrock, Acc. Chem. Res. 23 (1990) 158) oder ein komplexes Salz der Metalle Ru, Os oder W, wie z.B. $K_2RuCl_5$. Bei polaren Polymeren eignen sich außerdem Hydrate von $RuCl_3$ oder $OsCl_3$ als Initiatoren (W.J. Feast, D.B. Harrison, Polymer 32 (1991) 558). Weiter geeignet sind Zweikomponentenkatalysatoren wie $MCl_6$ + $R_xAlCl_{3-x}$ mit M = W oder Mo.

Bevorzugte Metallcarben-Komplexe sind:

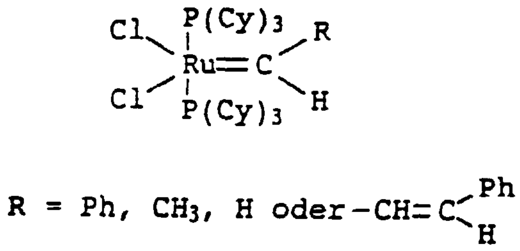

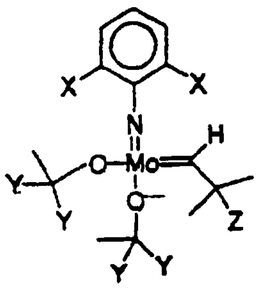

X = H oder $CH_3$-CH-$CH_3$
Y = $CH_3$, $CF_3$ oder Ph
Z = $CH_3$ oder Ph

(vgl. P. Schwab et al., Angew. Chemie, Intern. Ed. Engl., 34 (1995) 2039)

Die Metallcarben-Komplexe können auch in an einen oxidischen Träger, wie $SiO_2$, $Al_2O_3$ und $ZrO_2$, gebundener Form vorliegen.

Weiter eignen sich luft- oder wasserstabile Katalysatoren auf der Basis von Carbonylgruppen enthaltenden Mo-, Ru-, Os- oder W-Verbindungen mit einem Polyenliganden, wie sie z.B. in der WO 93/13171 beschrieben werden, beispielsweise $[(C_6H_6)Ru(CH_3CN)_2Cl]^+PF_6^-$, als Initiatoren.

Bei der Verwendung von Übergangsmetallcarbenkatalysatoren kann die Polymerisation bei Raumtemperatur und auch ohne die Zugabe von Cokatalysatoren oder die Bestrahlung mit Licht durchgeführt werden.

Bei der durch Übergangsmetallcarbene katalysierten RÖMP werden keine Abspaltprodukte oder flüchtige Reaktionsprodukte gebildet und der Katalysator selbst wird kovalent an das Polymer gebunden und auf diese Weise immobilisiert (vgl. F. Stelzer, J. Macromol. Sci. Pure. Appl. Chem A33 (1996) 941).

Als Photoinitiatoren und thermische Initiatoren sind photolabile und/oder thermolabile Ru- oder Os-Verbindungen, wie sie z.B. in der WO 96/23829) beschrieben werden, beispielsweise $[Ru(C_6H_6]_2$(Tosylat)$_2$, $RuCl_2$(Aren)$PR_3$, wobei Aren vorzugsweise für Cumol steht, $[Os(C_6H_6)Cl_2]_2$ oder Tantal(V)-Verbindungen wie $Ta[CH_2\text{-}Si(CH_3)_3]_3Cl_2$ bevorzugt.

Weiterhin enthalten die erfindungsgemäßen Zusammensetzungen zur Verbesserung der mechanischen Eigenschaften vorzugsweise einen oder mehrere Füllstoffe wie organische oder anorganische Partikel oder Fasern.

Bevorzugte anorganische partikuläre Füllstoffe sind amorphe kugelförmige Materialien auf der Basis von Mischoxiden aus $SiO_2$, $ZrO_2$ und/oder $TiO_2$ wie sie beispielsweise in der DE 40 29 230 A1 beschrieben werden, mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengröße von 0,01 bis 5 µm sowie röntgenopake Füllstoffe, wie Ytterbiumtrifluorid. Darüber hinaus können auch Glasfasern, Polyamid- oder Kohlenstoffasern als Füllstoffe eingesetzt werden.

Besonders bevorzugte Füllstoffe sind Mischungen aus (a) amorphen kugelförmigen Teilchen aus Siliciumdioxid und bis zu 20 mol-% eines Oxides mindestens eines Elements der Gruppen I, II, III und IV der Periodensystems mit einem Brechungsindex von 1,45 bis 1,58 und einer durchschnittlichen Primärpartikelgröße von 10 nm bis 10 µm und (b) Quarz-, Glaskeramik- oder Glaspulver oder deren Mischungen mit einem Brechungsindex von 1,45 bis 1,58 und einer durchschnittlichen Teilchengröße von 1,0 bis 15 µm.

Die Zusammensetzungen eignen sich besonders als Dentalmaterialien oder zur Herstellung von Dentalmaterialien, insbesondere als Prothesenkunststoffe, Materialien zur Herstellung künstlicher Zähne, Verblendmaterialien oder Füllungsmaterialien.

Die Zusammensetzungen enthalten vorzugsweise

(i) 1 bis 99 Gew.-%, vorzugsweise 10 bis 80 Gew.-% und besonders bevorzugt 20 bis 70 Gew.-% mindestens eines bicyclischen Ringsystems;
(ii) 0,001 bis 5 Gew.-%, vorzugsweise 0,1 bis 1,0 Gew.-% und ganz besonders bevorzugt 0,3 bis 0,8 Gew.-% mindestens eines RÖMP-Initiators und gegebenenfalls
(iii) 1 bis 95 Gew.-% Füllstoff.

Die Komponetne (i) enthält ihrerseits vorzugsweise 1 bis 60 Gew.-%, besonders bevorzugt 0,05 bis 20 Gew.-% eines RÖMP-Vernetzers, d.h. eines bicyclischen Ringsystems, das zwei oder mehr bicyclische Gruppen der oben definierten Art enthält.

Der Füllstoffgehalt wird maßgeblich durch die gewünschte Anwendung der Zusammensetzungen bestimmt. Im Falle von Prothesenkunststoffen und Materialien zur Herstellung künstlicher Zähne beträgt er vorzugsweise 0 bis 60 Gew.-%, bei Füllungsmaterialien (Kompositen) 50 bis 85 Gew.-%.

Außerdem können die Zusammensetzungen im Bedarfsfall weitere Hilfsstoffe wie Stabilisatoren, UV-Absorber, Farbstoffe, Pigmente und/oder Gleitmittel enthalten. Die Hilfstoffe werden ggf. in einer Menge von jeweils bis zu etwa 0,5 Gew.-% eingesetzt.

Geeignete Stabilisatoren sind zum Beispiel Hydrochinonmonomethylether oder 2,6-Di-tert.-butyl-4-methylphenol (BHT).

Ganz besonders bevorzugt sind Zusammensetzungen, die ausschließlich aus den Komponenten (i), (ii) und (iii) sowie ggf. den genannten Hilfsstoffen bestehen.

Im folgenden wird die Erfindung anhand von Ausführungsbeispielen näher erläutert.

## Beispiele

### Beispiel 1

Herstellung von Formkörpern durch ringöffnende Metathese-Polymerisation von Essigsäure-5-norbornen-2-ylester

In 10 g Essigsäure-5-norbornen-2-ylester mit einer Molmasse von 152,2 g/mol und einer Dichte von $\rho$ = 1,044 g/cm$^3$ werden als Initiator ca. 50 mg Rutheniumalkyliden-Komplex {Ru[P(Cyclohexyl)$_3$]Cl$_2$CHPh} gelöst. Die Monomer-Initiatormischung wird zur Aushärtung in Biegeprüffestigkeitsformen (25 x 2 x 2 mm; gemäß EN 24049) gegeben. Nach ca. 24 Stunden ist eine vollständige Aushärtung erfolgt. Der $^1$H-NMR-spektroskopisch bestimmte Restmonomergehalt (RM) liegt unter 1 Gew.-%, d.h. daß der Polymerisationsumsatz (U) über 99 % liegt ( U(%) = 100% - RM(%) ). Das ausgehärtete Material zeigt eine Biegefestigkeit von 37 MPa und einen Biege-E-Modul von 1400 MPa. Biegefestigkeit und Biege-E-Modul wurden in diesem und allen anderen Beispielen, wenn nicht anders angegeben, gemäß EN 24049 bestimmt. Mittels der Auftriebsmethode wurde die Dichte des Polymers zu $\rho$ = 1,112 g/cm$^3$ ermittelt, woraus sich ein Polymerisationsschrumpf von nur 6,1 Vol.-% ergibt. Der Polymerisationsschrumpf $\Delta V$ wurde aus der Differenz der Dichten von Monomer ($\rho M$) und Polymer ($\rho P$) gemäß der folgenden Formel $\Delta V$ (Vol.-%) = $[1 - (\rho_M/\rho_P)]$ berechnet.

Demgegenüber zeigt ein von der Molmasse etwa vergleichbares Butylmethacrylat (Molmasse 142,1 g/mol) bei der radikalischen Polymerisation einen Polymerisationsschrumpf von 15,7 Vol.-%. Aufgrund der beschriebenen Eigenschaften eignet sich das Material besonders als Prothesenkunststoff.

### Beispiel 2

Herstellung von Kompositformkörpern durch ringöffnende Metathese-Polymerisation (RÖMP) von Essigsäure-5-norbornen-2-ylester

Mittels eines Walzenstuhles (Modell Exakt, Exakt Apparatebau, Norderstedt, Deutschland) wird aus 35 g Essigsäure-5-norbornen-2-ylester und 65 g eines gemahlenen Bariumsilikatglases (Schott, Landshut, Deutschland) eine Kompositpaste mit einem Füllgrad von 65 Gew.-% hergestellt. Nach Zugabe von ca. 300 mg Rutheniumalkyliden-Komplex {Ru[P(Cyclohexyl)$_3$]Cl$_2$CHPh} wird die Paste schnell in Biegeprüffestigkeitsformen gegeben und dann innerhalb von 24 Stunden zur Aushärtung gebracht. Das ausgehärtete Material zeigt eine Biegefestigkeit von 65 MPa und einen Biege-E-Modul von 4800 MPa. Der aus der Dichtedifferenz zwischen Paste und ausgehärtetem Material berechnete Volumenschrumpf liegt bei nur 2,4 Vol.-%, so daß sich dieses Material besonders als Verblendmaterial eignet. Wird statt des oben genannten Rutheniumalkyliden-Komplexes ein Photoinitiator verwendet, wird ein Material erhalten, das sich besonders als Füllungsmaterial eignet.

## Patentansprüche

1. Zusammensetzung enthaltend

   (i) mindestens ein bicyclisches Ringsystem mit 6 bis 17 Kohlenstoff-, gegebenenfalls einem Heteroatom und mindestens einer endocyclischen Doppeldbindung; und

(ii) mindestens einen Initiator für die ringöffnende Metathese-Polymerisation;

mit der Maßgabe, daß das bicyclische Ringsystem nicht mit Methacrylatgruppen substituiert ist.

2. Zusammensetzung nach Anspruch 1, **dadurch gekennzeichnet,** daß das bicyclische Ringsystem ein Brücken-Ringsystem ist.

3. Zusammensetzung nach Anspruch 1 oder 2, **dadurch gekennzeichnet,** daß das bicyclische Ringsystem 1 bis 2 endocyclische Doppelbindungen enthält.

4. Zusammensetzung nach einem der Ansprüche 1 bis 3, **dadurch gekennzeichnet,** daß das bicyclische Ringsystem als Heteroatom Sauerstoff enthält.

5. Zusammensetzung nach einem der Ansprüche 1 bis 4, **dadurch gekennzeichnet,** daß das bicyclische Ringsystem (i) ein carbocyclischer oder heterocyclischer Bicyclo[x.y.z]kohlenwasserstoff ist, wobei x, y und z Werte von 1 bis 6 aufweisen können.

6. Zusammensetzung nach Anspruch 5, **dadurch gekennzeichnet,** daß x gleich 2, y gleich 2 und z gleich 1 ist.

7. Zusammensetzung nach Anspruch 6, **dadurch gekennzeichnet,** daß das bicyclische Ringsystem ein Bicyclo[2.2.1]heptenderivat ist.

8. Zusammensetzung nach Anspruch 7, **dadurch gekennzeichnet,** daß das bicyclische Ringsystem ein Bicyclo[2.2.1]heptenderivat der Formel I, II oder III oder eine dazu stereoisomere Verbindung ist

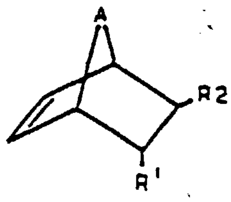

I

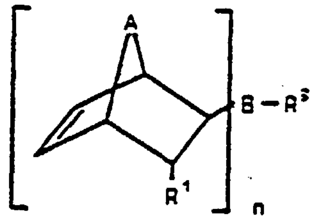

II

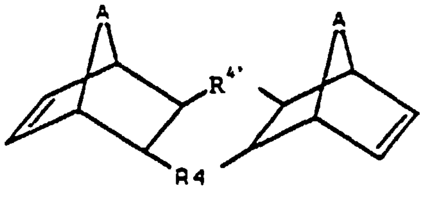

III

in denen n, A, B, $R^1$, $R^2$, $R^3$, $R^4$ und $R^{4'}$ unabhängig voneinander die folgenden Bedeutungen haben:

A = $-CH_2-$ oder -O-;

$R^1$, $R^2$ = -H; substituiertes oder unsubstituiertes $C_1$- bis $C_6$-Alkyl, $-C(=O)-OR^5$; $-O-C(=O)-R^5$; $-CH_2-O-C(=O)-R^5$; wobei $R^5$ für -H, substituiertes oder unsubstituiertes $C_1$- bis $C_6$-Alkyl oder Benzyl steht; oder $R^1$ und $R^2$ bilden zusammen mit den Kohlenstoffatomen, an die sie gebunden sind, ein ankondensiertes alicyclisches, heterocyclisches oder aromatisches Ringsystem mit 5 bis 12 Kohlenstoffatomen, das seinerseits durch $C_1$- bis $C_6$-Akylgruppen oder Benzyl substituiert sein kann;

B = $-CH_2-$ oder entfällt;
n = eine ganze Zahl von 1 bis 4;
$R^3$ = n-fach substituiertes $C_1$- bis $C_{10}$-Alkylen, C6- bis $C_{10}$-Arylen; -O-C(=O)-Phenylen-C(=O)-O-, $(-C(=O)-)_4(C_6H_2)$; 2,4,6-Trioxo-1,3,5-Triazinyl; $-O-C(=O)-(CH_2)_m-C(=O)-O-$; $-O-C(=O)-NH-(CH_2)_m-NH-C(=O)-O-$; wobei m = 1 bis 10 ist;
$R^4$, $R^{4'}$ = $C_1$- bis $C_{20}$-Alkylen, eine chemische Bindung oder -O-; oder $R^4$ und $R^{4'}$ bilden zusammen einen $>CH-CH_2-CH<$ Rest.

**9.** Zusammensetzung nach Anspruch 8, **dadurch gekennzeichnet,** daß die Variablen der Formeln I, II und III unabhängig voneinander die folgende Bedeutung haben:

A = $-CH_2-$;
$R^1$, $R^2$ = -H; $C_1$- bis $C_3$-Alkyl, $-C(=O)-OCH_3$; $-O-C(=O)-CH_3$; $-CH_2-O-C(=O)-CH_3$;
oder $R^1$ und $R^2$ bilden zusammen mit den Kohlenstoffatomen, an die sie gebunden sind, ein ankondensiertes alicyclisches oder aromatisches Ringsystem mit 5 bis 12 Kohlenstoffatomen, oder einen sauerstoffhaltigen Heterocyclus mit vier Kohlenstoff und einem Sauerstoffatom;
B = $-CH_2-$ oder entfällt;
n = eine ganze Zahl von 1 bis 3;
$R^3$ = n-fach substituiertes $C_1$- bis $C_3$-Alkylen; Phenylen, Naphthylen, -O-C(=O)-(Phenylen)-C(=O)-O-; $-O-C(=O)-(CH_2)_m-C(=O)-O-$;$-O-C(=O)-NH-(CH_2)_m-NH-C(=O)-O-$; wobei m = 1 bis 6 ist;
$R^4$, $R^{4'}$ = $C_1$- bis $C_{12}$-Alkylen, eine chemische Bindung oder -O-;
$R^5$ = $C_1$- bis $C_3$-Alkyl.

**10.** Zusammensetzung nach einem der Ansprüche 1 bis 9, **dadurch gekennzeichnet,** daß sie als Initiator für die ringöffnende Metathese-Polymerisation einen oder mehrere Metallcarben-Komplexe, $K_2RuCl_5$, ein oder mehrere Hydrate von $RuCl_3$ oder $OsCl_3$ und/oder als Zweikomponentenkatalysator $MCl_6 + R_xAlCl_{3-x}$ mit M = W oder Mo enthält.

**11.** Zusammensetzung nach Anspruch 10, **dadurch gekennzeichnet,** daß sie als Initiator einen Metallcarben-Komplex gemäß der Formel

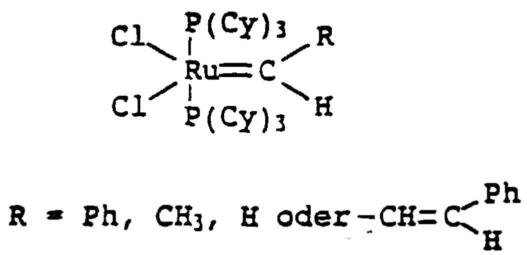

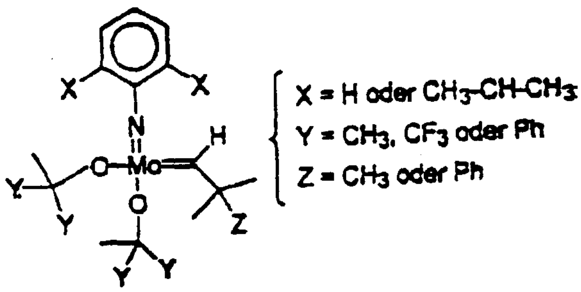

enthält.

**12.** Zusammensetzung nach einem der Ansprüche 1 bis 11 **dadurch gekennzeichnet,** daß sie

(i) 1 bis 99 Gew.-% des Bicyclischen Ringsystems; und

(ii) 0,001 bis 5 Gew.-% des Initiators enthält.

**13.** Zusammensetzung nach Anspruch 12, **dadurch gekennzeichnet,** daß sie zusätzlich

(iii) 1 bis 95 Gew.-% Füllstoff enthält.

**14.** Zusammensetzung nach Anspruch 12 oder 13, **dadurch gekennzeichnet,** daß die Komponente (i) 1 bis 60 Gew.-

% eines bicyclischen Ringsystems mit zwei oder mehr bicyclischen Gruppen enthält.

**15.** Dentalmaterial, gekennzeichnet durch einen Gehalt an einer Zusammensetzung gemäß einem der Ansprüche 1 bis 14.

**16.** Verwendung einer Zusammensetzung gemäß einem der Ansprüche 1 bis 15 als Dentalmaterial.

**17.** Verwendung nach Anspruch 16 als Prothesenkunststoff, Material zur Herstellung künstlicher Zähne, als Verblendmaterial oder als Füllungsmaterial.